# EUROPEAN PATENT SPECIFICATION

(11) **EP 3 549 533 B1**
(45) Date of publication and mention of the grant of the patent: **30.12.2020**
(21) Application number: 19168942.1
(22) Date of filing: 05.11.2013
(51) Int. Cl.: A61B 10/02

(54) **BIOPSY DEVICE HAVING INTEGRATED VACUUM**
BIOPSIEVORRICHTUNG MIT INTEGRIERTEM VAKUUM
DISPOSITIF DE BIOPSIE DOTÉ D'UN VIDE INTÉGRÉ

(43) Date of publication of application: 09.10.2019
(60) Divisional application: 20209785.3
(62) Document number(s) of the earlier application(s) in accordance with Art. 76 EPC: 13798425.8 / 3 065 643
(73) Proprietor: C.R. Bard, Inc., Franklin Lakes, NJ 07417 (US)
(72) Inventor: Schlarb, Rory M., Paradise Valley, NJ 85253 (US); Van Liere, Chad C., Phoenix, AZ 85042 (US)
(74) Representative: Hoffmann Eitle

(56) References cited:
- WO-A1-2013/158072
- US-A- 5 335 671
- US-A1- 2004 158 172
- US-A1- 2007 106 176
- US-A1- 2011 152 715

## Description

### BACKGROUND OF THE INVENTION

### 1. Field of the Invention

The present invention relates to biopsy devices, and, more particularly, to a handheld single insertion single sample (SISS) biopsy device having integrated vacuum.

### 2. Description of the Related Art

Biopsy devices typically include a power source and a sample retrieval mechanism. The sample retrieval mechanism may be in the form of a biopsy probe assembly configured with a sample retrieval opening for receiving tissue samples from the patient. Some practitioners that perform biopsy procedures prefer a self-contained handheld biopsy device over that of a large console system. There are essentially two types of self-contained handheld biopsy devices: the partially disposable biopsy device and the fully disposable biopsy device.

A typical partially disposable biopsy device has a reusable handheld driver to which a disposable probe is releasably attached. The reusable handheld driver is typically battery powered, and includes electrical motor drives and an on-board vacuum pump to aid in sample acquisition and/or retrieval. Often, such biopsy devices are configured for single insertion multiple sample (SIMS) procedures. The disposable probe is used on a single patient, and then discarded, while the handheld driver is retained for reuse.

A typical fully disposable biopsy device has one or more mechanical drives, such as spring/latch arrangements, which permit the biopsy device to be manually cocked and fired for tissue sample acquisition. Such simple biopsy devices often are configured to acquire a single sample per insertion. Also, many of the fully disposable biopsy devices do not have vacuum to assist in sample acquisition. While some attempts have been made to include a vacuum assist feature in a fully disposable biopsy device, the vacuum produced typically is not sufficient to approach the performance of that of a partially disposable biopsy device as described above. Also, in a typical fully disposable biopsy device having vacuum assist, such vacuum is generated simultaneously with movement of the cutting cannula to sever the tissue sample, and thus the vacuum may be of limited value in acquiring the tissue sample.

What is needed in the art is a biopsy device that may be fully disposable, while having efficient vacuum application to aid in sample acquisition, and which is configured to be easy to use.

### SUMMARY OF THE INVENTION

The present invention provides a biopsy device that is fully disposable, while having efficient vacuum application to aid in sample acquisition, and which is configured to be easy to use. The invention is directed to a biopsy device as defined in claim 1.

The disclosure includes a biopsy device having a housing body, a cannula assembly, a vacuum source and a trigger slide assembly. The housing body defines a longitudinal axis. The cannula assembly has a first elongate cannula having a first side wall configured to define a first lumen and an elongate side opening that extends through the first side wall. A second elongate cannula is coaxial with the first elongate cannula. The second elongate cannula has a second side wall configured to define a second lumen and a cutting edge. The second side wall has a vacuum side port in fluid communication with the second lumen. A vacuum source is positioned in the housing body. The vacuum source has a chamber side wall having a chamber vacuum port. A seal is interposed in sealing engagement between the chamber vacuum port and the second elongate cannula. A trigger slide assembly is coupled to the housing body, and is coupled to the cannula assembly. The trigger slide assembly is configured to move the second elongate cannula to align the vacuum side port of the second elongate cannula with the chamber vacuum port of the vacuum source to supply vacuum from the vacuum source to the second lumen of the second elongate cannula.

The disclosure further includes a biopsy device that includes a housing body defining a longitudinal axis. A first cannula assembly has a first elongate cannula that extends along the longitudinal axis. The first elongate cannula has a first side wall configured to define a first lumen and has an elongate side opening that extends through the first side wall. A second cannula assembly includes a second cannula body coupled to a second elongate cannula that is slidably received in the first lumen of the first elongate cannula. The second cannula body has a drive tab and a proximal latch mechanism configured to releasably latch the second elongate cannula in a retracted position. The second elongate cannula has a second side wall having a second lumen, and has a vacuum side port that extends through the second side wall and is in fluid communication with the second lumen. A cannula drive spring is configured to compress when the proximal latch mechanism is moved to the retracted position and configured to decompress to propel the second cannula assembly in a distal direction when the proximal latch mechanism is released from the retracted position. A vacuum source is positioned in the housing body. The vacuum source is configured to store a vacuum. The vacuum source includes a chamber side wall defining a volume. The chamber side wall has a chamber vacuum port. A vacuum seal is interposed in sealing engagement between the chamber vacuum port and the second elongate cannula. A trigger slide assembly has a slider body coupled to the housing body and coupled to the drive tab of the second cannula body. The trigger slide assembly is configured such that: a first proximal movement of the slider body in a proximal direction retracts the second cannula assembly a first distance to latch the proximal latch mechanism of the second cannula assembly in the retracted position and compress the cannula drive spring; a second proximal movement of the slider body in the proximal direction retracts the second cannula assembly a second distance cumulative with the first distance to radially align the vacuum side port of the second elongate cannula with the chamber vacuum port of the vacuum source to supply vacuum from the vacuum source to the second lumen of the second elongate cannula; and a third movement of the slider body releases the proximal latch mechanism such that the cannula drive spring decompresses to propel the second elongate cannula of the second cannula assembly in a distal direction.

The disclosure further includes a biopsy device. The biopsy device includes a housing body defining a longitudinal axis. A cannula assembly has an actuator body and an elongate cannula affixed to the actuator body. The elongate cannula has a side wall defining a lumen, and has a vacuum side port that extends through the side wall and is in fluid communication with the lumen. The actuator body has a drive tab and a proximal latch mechanism. The proximal latch mechanism is configured to selectively engage the housing body to releasably latch the cannula assembly in a retracted position. A cannula drive spring is coupled between the housing body and the actuator body. The cannula drive spring is configured to be compressed when the proximal latch mechanism is moved to the retracted position and configured to decompress to propel the elongate cannula in a distal direction when the proximal latch mechanism is released from the retracted position. A vacuum source is coupled to the housing body and is configured to store a vacuum. The vacuum source includes a vacuum chamber housing having a chamber open end, a chamber end wall, a chamber side wall extending between the chamber open end and the chamber end wall, and a chamber vacuum port. The chamber side wall has a perimeter defining a U-shaped area in cross-section that extends longitudinally between the chamber open end and the chamber end wall to define a U-shaped volume. A trigger slide assembly has a slider body operatively coupled to the drive tab of the cannula assembly and to the vacuum source.

Also disclosed is a biopsy device that includes a housing body and a cannula assembly having an actuator body and an elongate cannula affixed to the actuator body. The elongate cannula has a side wall defining a lumen, and has a vacuum side port that extends through the side wall and in fluid communication with the lumen. The actuator body has a drive tab and a proximal latch mechanism. The proximal latch mechanism is configured to selectively engage the housing body to releasably latch the cannula assembly in a retracted position. A cannula drive spring is coupled between the housing body and the actuator body. The cannula drive spring is configured to be compressed when the proximal latch mechanism is moved to the retracted position and is configured to decompress to propel the elongate cannula in a distal direction when the proximal latch mechanism is released from the retracted position. A vacuum source is coupled to the housing body and is configured to store a vacuum. The vacuum source includes a vacuum chamber housing having a chamber side wall having a chamber vacuum port. A trigger slide assembly has a slider body slidably coupled to the housing body, and operatively coupled to the drive tab of the actuator body of the cannula assembly. The trigger slide assembly is configured such that: a first proximal movement of the slider body retracts the cannula assembly a first distance to latch the cannula assembly in the retracted position and to compress the cannula drive spring; a second proximal movement of the slider body retracts the cannula assembly a second distance cumulative with the first distance to align the vacuum side port of the elongate cannula with the chamber vacuum port of the vacuum source to supply vacuum from the vacuum source to the second lumen of the elongate cannula; and a third movement of the slider body in a distal direction releases the proximal latch mechanism such that the cannula drive spring decompresses to propel the cannula assembly in the distal direction.

### BRIEF DESCRIPTION OF THE DRAWINGS

The above-mentioned and other features and advantages of this invention, and the manner of attaining them, will become more apparent and the invention will be better understood by reference to the following description of an embodiment of the invention taken in conjunction with the accompanying drawings, wherein:
Fig. 1 is a perspective view of a biopsy device in accordance with an embodiment of the present invention;
Fig. 2 is a side view of the biopsy device of Fig. 1;
Fig. 3 is an exploded view of the biopsy device of Fig. 1;
Fig. 4 is a section view of the biopsy device of Fig. 2, taken along plane 4-4;
Fig. 5 is a section view corresponding to the section view of Fig. 4, showing the latch mechanisms of the inner cannula assembly and the vacuum source in their respective latched (primed) positions;
Fig. 6 is a section view of the biopsy device of Fig. 2, taken along plane 6-6;
Fig. 7 is a section view of the biopsy device of Figs. 1 and 2, taken along the plane 7-7 of Fig. 1, and showing the vacuum source, the inner cannula assembly, the vacuum prime handle mechanism, and the slider body of the trigger slide assembly in their respective home positions;
Fig. 8 is a section view corresponding to the section of Fig. 7, showing the vacuum source in the primed position and the vacuum prime handle mechanism fully retracted;
Fig. 9 is a section view corresponding to the section of Fig. 7, showing the vacuum source in the primed position and the vacuum prime handle mechanism in the return primed position;
Fig. 10 is a section view corresponding to the section of Fig. 7, showing the slider body of the trigger slide assembly in a first retracted primed position, and the inner cannula assembly in the primed (cocked) position;
Fig. 11 is an enlarged view of a portion of the section view of Fig. 10 showing the vacuum source with the chamber vacuum port closed by the cutting cannula side wall;
Fig. 12 is a section view corresponding to the section of Fig. 7, showing the slider body of the trigger slide assembly and the inner cannula assembly in a further retracted, vacuum application position;
Fig. 13 is an enlarged view of a portion of the section view of Fig. 12, showing the vacuum source with the chamber vacuum port open and in fluid communication with the vacuum side port of the cutting cannula side wall;
Fig. 14 is a section view corresponding to the section of Fig. 7, showing the inner cannula assembly in the primed (cocked) position, and with the slider body of the trigger slide assembly returned to the home position; and
Fig. 15 is an enlarged view of a portion of the section view of Fig. 14, showing the slider body of the trigger slide assembly ready to deflect the cannula latch mechanism downwardly for release upon a distal movement of the slider body beyond its home position.

Corresponding reference characters indicate corresponding parts throughout the several views. The exemplification set out herein illustrates an embodiment of the invention and such exemplification is not to be construed as limiting the scope of the invention in any manner.

### DETAILED DESCRIPTION OF THE INVENTION

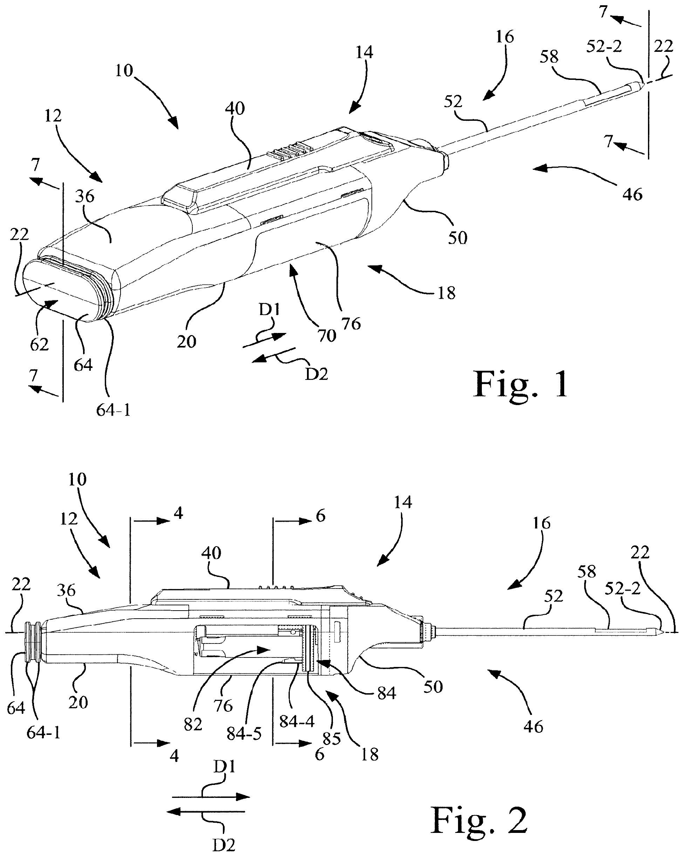

Referring now to the drawings, and more particularly to Figs. 1-7, there is shown a biopsy device 10 in accordance with an embodiment of the present invention. Biopsy device 10 is configured as a single insertion single sample (SISS) biopsy device, which is fully disposable.

As shown in Figs. 1-3, biopsy device 10 generally includes a housing 12, a trigger slide assembly 14, a cannula assembly 16, and a vacuum source 18. In describing biopsy device 10, for convenience, reference will be made to distal direction D1 and proximal direction D2.

Housing 12 includes a housing body 20 that defines a longitudinal axis 22. Housing body 20 has a proximal end wall 24, a distal end wall 26, an upper surface 28, and an interior wall 30. Interior wall 30 is located between proximal end wall 24 and distal end wall 26. Interior wall 30 separates the housing body 20 into a proximal chamber 32 and a distal chamber 34. A chamber cover 36 is removably attached to housing body 20 to enclose proximal chamber 32.

Proximal end wall 24 has a pair of handle link openings 24-1, 24-2.

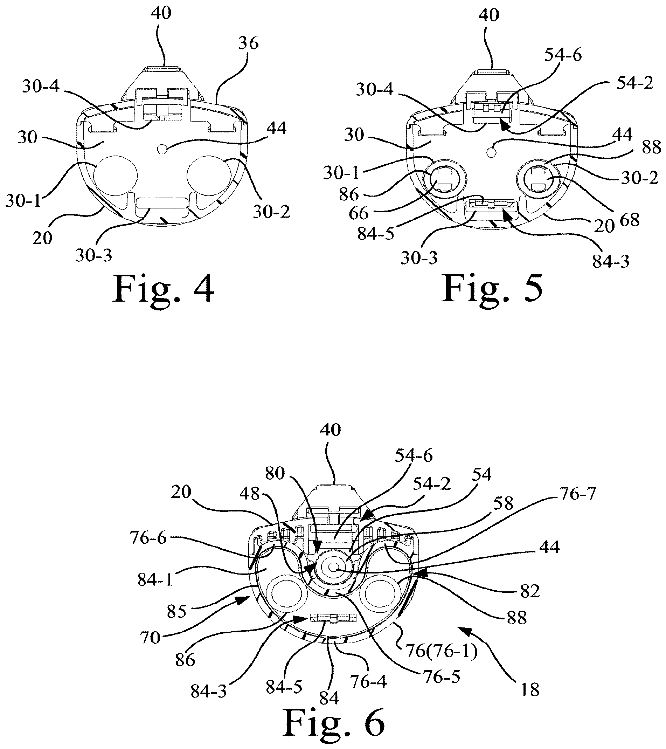

Referring also to Figs. 4 and 5, interior wall 30 has two piston link openings 30-1, 30-2, a piston latch opening 30-3, and a cannula latch opening 30-4.

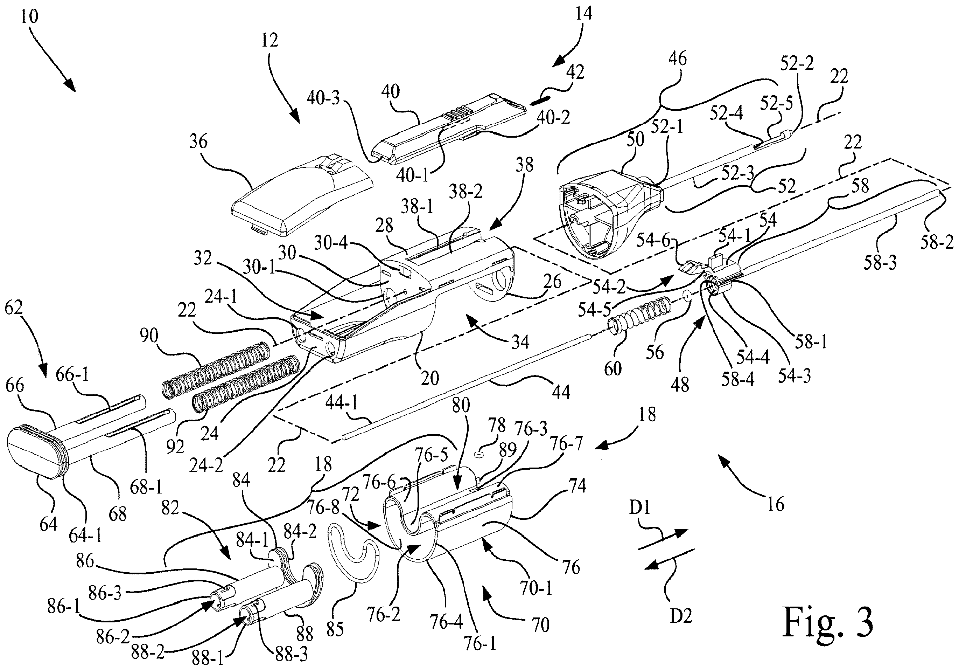

As best shown in Fig. 3, upper surface 28 of the housing body 20 has an elongate slide slot 38 that defines a first slide slot edge 38-1 and a second slide slot edge 38-2. Second slide slot edge 38-2 is spaced apart from the first slide slot edge 38-1 in a direction perpendicular to the longitudinal axis 22.

Trigger slide assembly 14 is coupled to housing body 20 at elongate slide slot 38. Trigger slide assembly 14 includes a slider body 40 having a pair of opposed channels 40-1, 40-2 configured to respectively receive the first slide slot edge 38-1 and the second slide slot edge 38-2 of the elongate slide slot 38 of the housing body 20. Trigger slide assembly 14 is biased in the distal direction D1 by a biasing spring 42.

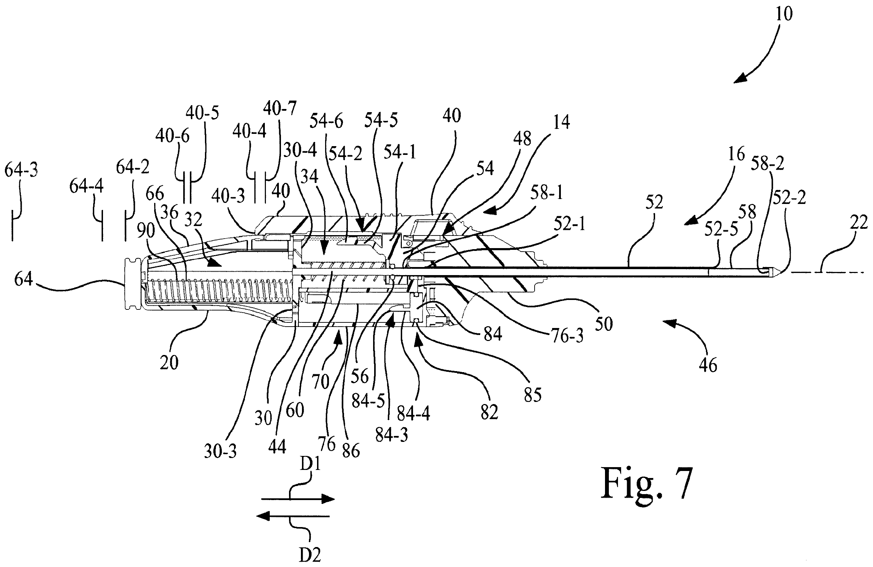

Referring also to Figs. 7-10, 12 and 14, and using a proximal end 40-3 of slider body 40 as a point of reference, slider body 40 has four positions, namely: a home position 40-4, a first proximal position 40-5, a second proximal position 40-6, and a distal-most position 40-7. The four positions of slider body 40 will be described in more detail below.

As best shown in Fig. 3, cannula assembly 16 includes a cannula support rod 44, an outer cannula assembly 46, and an inner cannula assembly 48.

Cannula support rod 44 is disposed in housing body 20 on and co-extensive with the longitudinal axis 22. Cannula support rod 44 has a proximal end 44-1 connected to the interior wall 30 of the housing body 20. Cannula support rod 44 provides radial support along longitudinal axis 22 for the stationary outer cannula assembly 46 as well as the movable inner cannula assembly 48.

Outer cannula assembly 46 has an end cap body 50 and an elongate outer cannula 52 that extends along the longitudinal axis 22. End cap body 50 is connected to housing body 20 at distal end wall 26 of the housing body 20. As such, outer cannula 52 is stationary relative to housing body 20. Outer cannula 52 is coaxial with cannula support rod 44, and is received over a portion of cannula support rod 44.

Outer cannula 52 has an end 52-1, a penetrating tip 52-2, and a side wall 52-3 that extends between the end 52-1 and penetrating tip 52-2. The end 52-1 is affixed to end cap body 50. Side wall 52-3 is configured to define a lumen 52-4 and has an elongate side opening 52-5 that extends through side wall 52-3. Elongate side opening 52-5 is thus in fluid communication with the lumen 52-4. Elongate side opening 52-5 is configured to receive a tissue sample during a biopsy procedure.

Inner cannula assembly 48 includes an actuator body 54, a cannula support rod seal 56, and an elongate cutting cannula 58.

Referring also to Fig. 7 in conjunction with Fig. 3, actuator body 54 has a drive tab 54-1, a proximal latch mechanism 54-2, a cannula mount 54-3 and proximal axial bore 54-4. Drive tab 54-1 is configured to engage slider body 40 of trigger slide assembly 14 for longitudinal movement therewith, e.g., in the proximal direction D2.

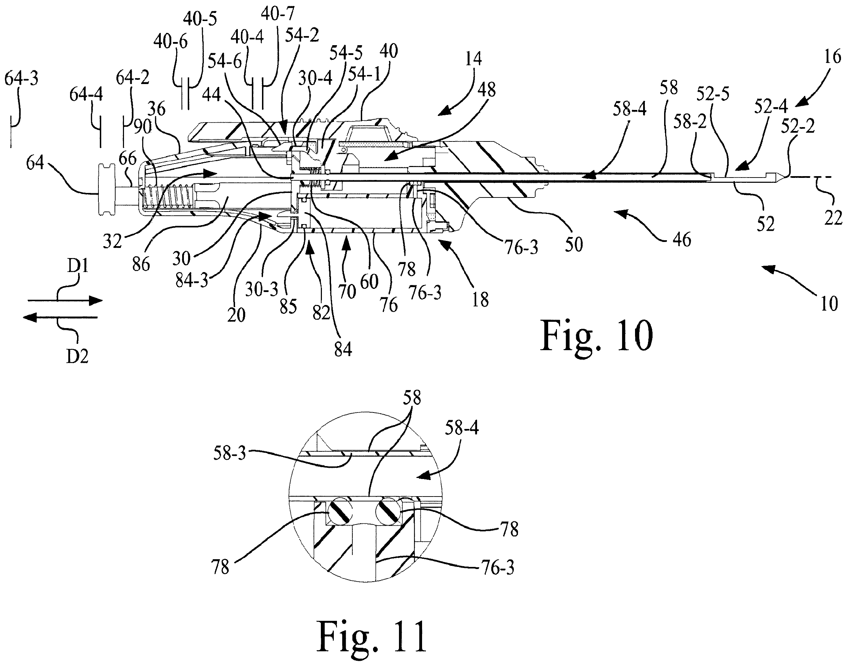

Referring also to Fig. 10, together, proximal latch mechanism 54-2 of actuator body 54 and cannula latch opening 30-4 of interior wall 30 of housing body 20 form a snap featured detent. Proximal latch mechanism 54-2 is configured to pass through the cannula latch opening 30-4 of interior wall 30 of housing body 20, to thereby releasably latch the elongate cutting cannula 58 in a retracted (primed, or sometimes referred to as cocked) position. More particularly, proximal latch mechanism 54-2 is in the form of a cantilever arm 54-5 having at its free end a latch head 54-6 configured to catch a portion of interior wall 30 (see also Fig. 5) adjacent cannula latch opening 30-4 in proximal chamber 32 to releasably latch the elongate cutting cannula 58 in the retracted (primed, or cocked) position.

Cutting cannula 58 is coaxial with cannula support rod 44 and outer cannula 52. More particularly, in the present embodiment, cutting cannula 58 is radially interposed between cannula support rod 44 and outer cannula 52, with cutting cannula 58 being slidably received in the lumen 52-4 of outer cannula 52 and slidably receiving cannula support rod 44.

As best shown in Fig. 3, cutting cannula 58 has an end portion 58-1, a distal cutting edge 58-2, and a side wall 58-3 (see also Fig. 11) that extends between end portion 58-1 and distal cutting edge 58-2. End portion 58-1 is affixed to the cannula mount 54-3, with end portion 58-1 being in fluid communication with the proximal axial bore 54-4 of actuator body 54.

Side wall 58-3 defines an inner lumen 58-4. Inner lumen 58-4 is slidably received over the cannula support rod 44. Cannula support rod seal 56, in the form of a rubber O-ring, is positioned within proximal axial bore 54-4 of the actuator body 54. Cannula support rod 44 is received through the aperture of cannula support rod seal 56, with cannula support rod seal 56 being radially interposed between actuator body 54 and cannula support rod 44. As such, cannula support rod seal 56 is configured to be axially stationary within proximal axial bore 54-4 of actuator body 54, while being axially movable along the cannula support rod 44 with actuator body 54.

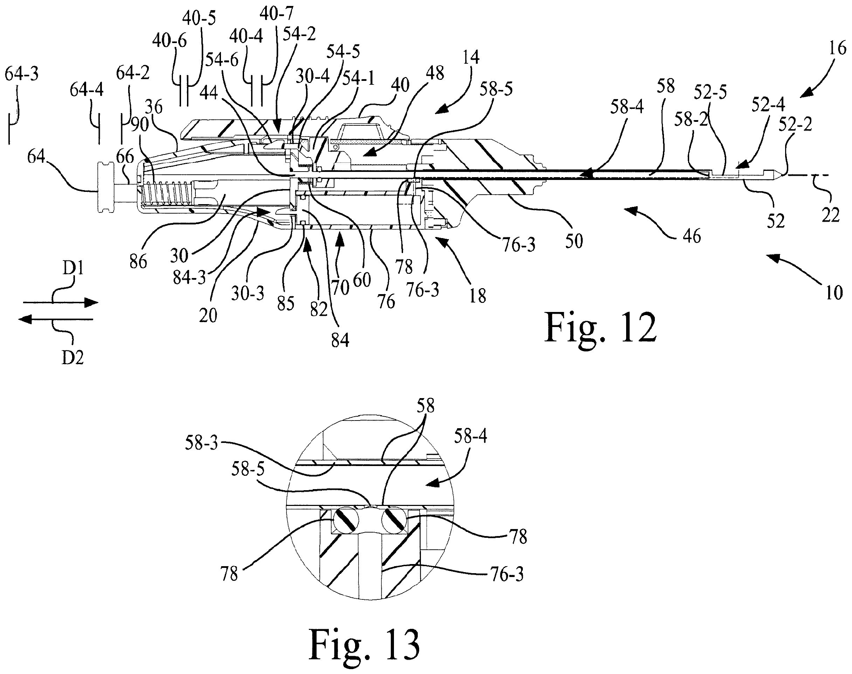

Referring also to Figs. 12 and 13, cutting cannula 58 further includes a vacuum side port 58-5 that extends through the side wall 58-3 at the end portion 58-1 and is in fluid communication with inner lumen 58-4. With cutting cannula 58 positioned in lumen 52-4 of outer cannula 52, vacuum side port 58-5 is further in fluid communication with a distal portion of outer cannula 52 that includes elongate side opening 52-5.

Referring again also to Figs. 3 and 7-9, a cannula drive spring 60, e.g., in the form of a coil spring, is positioned in distal chamber 34 of housing body 20, between interior wall 30 of housing body 20 and the actuator body 54 of the inner cannula assembly 48. Referring also to Figs. 10, 12, and 14, cannula drive spring 60 is configured to compress to store energy when the inner cannula assembly 48 is moved in the proximal direction D2 to the retracted position, at which time latch head 54-6 of proximal latch mechanism 54-2 passes through the cannula latch opening 30-4 of interior wall 30 of housing body 20 to thereby releasably latch inner cannula assembly 48 (including elongate cutting cannula 58) in a retracted position. Likewise, cannula drive spring 60 is configured to decompress to release the stored energy to propel the inner cannula assembly 48 in a distal direction D1 when proximal latch mechanism 54-2 is released from its latched state.

As best shown in Figs. 3 and 7-9, supplemental to vacuum source 18 is a vacuum prime handle mechanism 62. Vacuum prime handle mechanism 62 is configured to prime, i.e., charge, vacuum source 18 with a supply of vacuum. Vacuum prime handle mechanism 62 includes a handle base 64 and a pair of elongate handle links 66, 68 that extend in the distal direction D1 from handle base 64. Handle base 64 may include one or more perimetrical gripping ridges 64-1.

The pair of elongate handle links 66, 68 is positioned to pass through the respective pair of handle link openings 24-1, 24-2 of the proximal end wall 24 and into the proximal chamber 32. Each of the pair of elongate handle links 66, 68 has a respective longitudinal slot 66-1, 68-1. Each of the pair of elongate handle links 66, 68 also is configured to pass through a respective link opening of the piston link openings 30-1, 30-2 in the interior wall 30 of the housing body 20 to engage vacuum source 18, as will be more fully described below.

Referring again to Figs. 3 and 6, vacuum source 18 includes a vacuum chamber housing 70 having a chamber open end 72, a chamber end wall 74, and a chamber side wall 76. Chamber side wall 76 has a perimeter 76-1 corresponding to exterior surface 70-1 defining a U-shaped area in cross-section that extends longitudinally between the chamber open end 72 and the chamber end wall 74 to define a U-shaped volume 76-2. The U-shape construction of chamber side wall 76 facilitates the ability for inner cannula assembly 48 to be moveably tucked within the same footprint as that of vacuum source 18, thereby efficiently utilizing space within biopsy device 10.

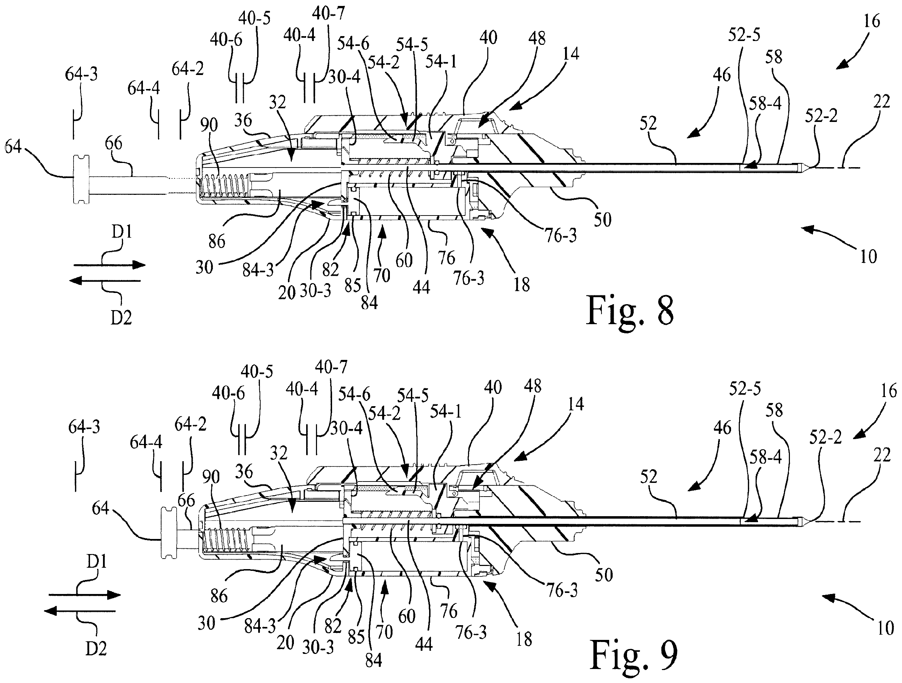

Referring also to Figs. 8-13, chamber side wall 76 has a chamber vacuum port 76-3. Referring particularly to Figs. 11 and 13, vacuum side port 58-5 of elongate cutting cannula 58 is configured for selective fluid engagement with chamber vacuum port 76-3 of vacuum chamber housing 70. Chamber vacuum port 76-3 is configured as an elevated protrusion of the chamber side wall 76, with the elevated protrusion having an aperture that extends through the elevated protrusion of the chamber side wall 76 to the U-shaped volume 76-2. A vacuum chamber housing seal 78, in the form of a rubber O-ring, is interposed in sealing engagement between chamber vacuum port 76-3 of vacuum chamber housing 70 and elongate cutting cannula 58. When chamber vacuum port 76-3 of vacuum chamber housing 70 and vacuum side port 58-5 of elongate cutting cannula 58 are radially aligned, vacuum chamber housing seal 78 is interposed in sealing engagement between chamber vacuum port 76-3 of vacuum chamber housing 70 and vacuum side port 58-5 of elongate cutting cannula 58 so as to facilitate the establishment of vacuum in inner lumen 58-4 of cutting cannula 58.

Referring particularly to Figs. 3 and 6, chamber side wall 76 has a first U-shaped wall section 76-4, a second U-shaped wall section 76-5, a first inverted U-shaped wall section 76-6 and a second inverted U-shaped wall section 76-7. Second U-shaped wall section 76-5 is smaller than the first U-shaped wall section 76-4 to define an upper elongate recessed trough 80 at exterior surface 70-1 at an upper portion of chamber side wall 76 of the vacuum chamber housing 70. Second U-shaped wall section 76-5 is located between the first inverted U-shaped wall section 76-6 and the second inverted U-shaped wall section 76-7 around perimeter 76-1 of the upper portion of chamber side wall 76.

As best shown in Figs. 3 and 6, elongated recessed trough 80 is configured to receive inner cannula assembly 48, including actuator body 54 and cutting cannula 58. More particularly, actuator body 54 includes a lower curved surface that is radially supported along the longitudinal extent of recessed trough 80, and cutting cannula 58 is received in elongated recessed trough 80 without contacting chamber side wall 76. Referring again also to Figs. 10-13, chamber vacuum port 76-3 extends outwardly from chamber side wall 76 from within elongate recessed trough 80 in a direction toward cutting cannula 58.

Referring to Figs. 2, 3, 6, and 7, vacuum source 18 further includes a vacuum plunger mechanism 82. Vacuum plunger mechanism 82 includes a U-shaped piston 84 positioned in U-shaped volume 76-2. U-shaped piston 84 has a proximal surface 84-1 and a distal surface 84-2. A chamber seal 85, in the form of a U-shaped rubber O-ring, is configured for sealing engagement between an interior surface 76-8 of the chamber side wall 76 and the U-shaped piston 84.

Referring to Figs. 2 and 5-10, a piston latch mechanism 84-3 extends in the proximal direction D2 from proximal surface 84-1, and is in the form of a cantilever arm 84-4 having at its free end a latch head 84-5. Together, piston latch mechanism 84-3 of U-shaped piston 84 and piston latch opening 30-3 of interior wall 30 of housing body 20 form a snap featured detent.

Referring particularly to Figs. 8 and 9, when U-shaped piston 84 is moved, i.e., retracted, in the proximal direction D2 to prime vacuum source 18, vacuum is accumulated in vacuum chamber housing 70 and thus vacuum chamber housing 70 is considered to be primed. Piston latch mechanism 84-3 is configured to pass through the piston latch opening 30-3 of interior wall 30 of housing body 20 to thereby releasably latch U-shaped piston 84 in a retracted (primed) position. More particularly, piston latch mechanism 84-3 latch head 84-5 is configured to catch a portion of interior wall 30 adjacent piston latch opening 30-3 in proximal chamber 32 to releasably latch U-shaped piston 84 in the retracted (primed) position.

Referring to Figs. 8-10, 12 and 14, when vacuum source 18 is primed, vacuum chamber housing 70 in conjunction with U-shaped piston 84 define approximately 20 cubic centimeters of vacuum storage, and with vacuum pressure at full charge in the range of -5.0 psi to -6.0 psi (-34.4k Pa to -41.4k Pa).

Referring again to Fig. 3, vacuum source 18 is configured with a check valve 89 to allow vacuum to accumulate in vacuum chamber housing 70 on retraction of U-shaped piston 84 in the proximal direction D2, but also to equalize pressure when U-shaped piston 84 is released and moved in the distal direction D1 toward chamber end wall 74 in preparation for a vacuum re-prime operation. Check valve 89 is configured to facilitate fluid flow in only one direction, and may be, for example, in the form of a ball/spring valve. As shown in Fig. 3, check valve 89 is located on chamber end wall 74 of vacuum chamber housing 70. Alternatively, it is contemplated that check valve 89 may be built into U-shaped piston 84.

Referring to Figs. 3, 5, 6, 8-10, 12 and 14, a pair of piston link rods 86, 88 extends proximally from proximal surface 84-1 of the U-shaped piston 84. Each of the pair of piston link rods 86, 88 has a respective proximal end 86-1, 88-1 and a bore 86-2, 88-2 extending distally from the proximal end 86-1, 88-1. Each of the pair of piston link rods 86, 88 is configured to pass through a respective piston link opening 30-1, 30-2 (see Figs. 4 and 5) in interior wall 30 of housing body 20 and into proximal chamber 32 (see Figs. 8-10). Each of a pair of bias springs 90, 92 is respectively received over the pair of elongate handle links 66, 68, and is positioned between the proximal end wall 24 of the housing body 20 and a respective proximal end 86-1, 88-1 of the pair of piston link rods 86, 88. A pair of joining features 86-3, 88-3, e.g., tabs, clips, pins, etc., may be affixed to, or integral with, a respective piston link rod of the pair of piston link rods 86, 88 of vacuum plunger mechanism 82 and is configured to be slidably engaged within a respective longitudinal slot 66-1, 68-1 of a respective elongate handle link 66, 68 of vacuum prime handle mechanism 62. In the present embodiment, each of the pair of joining features 86-3, 88-3 is formed as a part of a snap tab located on a respective piston link rod of the pair of piston link rods 86, 88 and is configured to engage a respective longitudinal slot 66-1, 68-1 of a respective elongate handle link 66, 68 of vacuum prime handle mechanism 62. Alternatively, it is contemplated that joining features 86-3, 88-3 may be a separate fastener, e.g., a pin.

The operation of biopsy device 10 now will be described with primary reference to Figs. 7-15, and with overall reference to Fig. 3.

Fig. 7 shows biopsy device 10 with all components in their respective home position. More particularly, the home position 40-4 of slider body 40 is the position to which slider body 40 will always return when no external force is applied to slider body 40. Also, a home position 64-2 of handle base 64 of vacuum prime handle mechanism 62 is the position where handle base 64 is immediately adjacent to the proximal-most portion of housing body 20.

Referring to Fig. 8, to ready biopsy device 10 for performing a biopsy procedure, vacuum source 18 is primed, i.e., vacuum is stored in vacuum chamber housing 70. To prime vacuum source 18, the user pulls handle base 64 in the proximal direction D2 to its proximal-most position 64-3, which in turn causes a proximal retraction in the proximal direction D2 of vacuum plunger mechanism 82 to a fully retracted (prime) position.

More particularly, a proximal movement of handle base 64 toward proximal-most position 64-3 results in a proximal movement of the handle links 66, 68. Since handle links 66, 68 are slidably coupled to piston link rods 86, 88, the proximal movement of handle links 66, 68 result in a proximal movement of vacuum plunger mechanism 82 to the vacuum primed position, wherein when handle base 64 reaches proximal-most position 64-3, piston latch mechanism 84-3 passes through the piston latch opening 30-3 of interior wall 30 of housing body 20 and releasably latches U-shaped piston 84 in a retracted (primed) position. Simultaneously, springs 90, 92 are compressed.

Referring to Fig. 9, after priming vacuum source 18, handle base 64 is moved in the distal direction D1 to the proximal intermediate position 64-4. Because handle links 66, 68 are fixedly attached to handle base 64, the movement of handle base 64 also results in movement of handle links 66, 68. However, since handle links 66, 68 are slidably joined to piston link rods 86, 88 via longitudinal slot 66-1, 68-1, and while U-shaped piston 84 is latched in a retracted (primed) position, handle links 66, 68 are free to move in the distal direction D1 within piston link rods 86, 88 until handle links 66, 68 are collapsed into piston link rods 86, 88 to a point of resistance. The point of resistance may be defined, for example, by the length of longitudinal slots 66-1, 68-1, or alternatively, when the distal end of handle links 66, 68 encounter the proximal surface 84-1 of U-shaped piston 84 within piston link rods 86, 88.

Thus, with handle base 64 at proximal intermediate position 64-4, distal movement of handle base 64 is restricted. However, if it is desired to re-prime and/or de-prime vacuum source 18, the user may do so by firmly applying pressure (e.g., a firm bump with the hand) in the distal direction D1 to handle base 64 to overcome the latch force of piston latch mechanism 84-3, and with springs 90, 92 decompressing, U-shaped piston 84 is moved back to its home position (see Fig. 7).

After priming vacuum source 18 (Fig. 9), biopsy device 10 is ready for insertion of cannula assembly 16 into the tissue of the patient for positioning at the biopsy site. In particular, cannula assembly 16 is inserted into the patient such that elongate side opening 52-5 of outer cannula 52 is positioned adjacent the tissue to be sampled at the biopsy site.

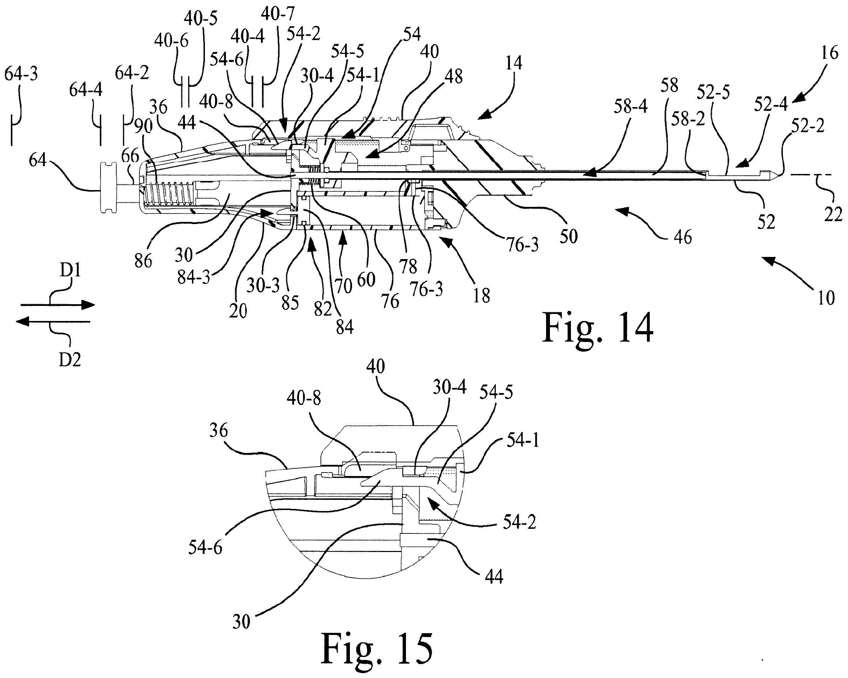

Referring to Figs. 10, 12 and 14, next slider body 40 of trigger slide assembly 14 will be moved sequentially from home position 40-4 to first proximal position 40-5 (Fig. 10), then to second proximal position 40-6 (Fig. 12), then back to home position 40-4 (Fig. 9), and then to distal-most position 40-7 (see Fig. 14), to effect a tissue sample capture sequence.

Again, home position 40-4 (see, e.g., Fig. 9) of slider body 40 is the position to which slider body 40 will always return when no external force is applied to slider body 40. Thus, if at any time slider body 40 is released by the user, either intentionally or inadvertently, slider body 40 will always return to the home position 40-4 and the sequence can be resumed from home position 40-4.

Referring to Fig. 10, first proximal position 40-5 is the position of slider body 40 where a prime (cocking) of inner cannula assembly 48, including cutting cannula 58, occurs. As slider body 40 is moved in the proximal direction D2, slider body 40 engages drive tab 54-1 of actuator body 54 of inner cannula assembly 48 to which cutting cannula 58 is fixedly attached. Thus, a longitudinal movement of slider body 40 in the proximal direction D2 will result in a corresponding movement is proximal direction D2 of inner cannula assembly 48 having cutting cannula 58.

This proximal movement of the slider body to first proximal position 40-5 retracts the inner cannula assembly 48 a first distance to latch the proximal latch mechanism 54-2 of the elongate cutting cannula 58 in a retracted (primed, or cocked) position and compresses the cannula drive spring 60. More particularly, proximal latch mechanism 54-2 passes through the cannula latch opening 30-4 of interior wall 30 of housing body 20 to releasably latch the elongate cutting cannula 58 in the retracted (primed, or cocked) position. In the primed (cocked) position, elongate cutting cannula 58 has been retracted to open elongate side opening 52-5 of outer cannula 52.

Referring also to Fig. 11, when slider body 40 of trigger slide assembly 14 is in any position other than in the second proximal position 40-6, cutting cannula 58 will block chamber vacuum port 76-3 of vacuum chamber housing 70, and thus prevent the supply of vacuum stored in vacuum chamber housing 70 from escaping.

Thus, referring now to Fig. 12, in order to apply vacuum to elongate side opening 52-5 of outer cannula 52 during the biopsy procedure, slider body 40 is moved to the second proximal position 40-6, which is spaced a distance more proximal than first proximal position 40-5. Thus, the second proximal movement of the slider body to second proximal position 40-6 retracts the inner cannula assembly 48 a second distance cumulative with the first proximal distance associated with first proximal position 40-5, so as to radially align vacuum side port 58-5 of elongate cutting cannula 58 with the chamber vacuum port 76-3 of the vacuum source 18 (see Fig. 13) such that vacuum is transferred from vacuum chamber housing 70 of vacuum source 18 to the inner lumen 58-4 of cutting cannula 58, and in turn to elongate side opening 52-5 of outer cannula 52, so as to apply vacuum to the tissue adjacent elongate side opening 52-5 so as to draw the tissue into elongate side opening 52-5 prior to releasing cutting cannula 58.

The user will maintain slider body 40 of trigger slide assembly 14 at the second proximal position 40-6 only so long as deemed necessary to establish the vacuum at elongate side opening 52-5 of outer cannula 52 and draw the tissue to be sampled into elongate side opening 52-5 of outer cannula 52. This time period may be, for example, from 0.5 to 2 seconds, as determined by the practitioner. The user will then release slider body 40, and biasing spring 42 will return slider body 40 to home position 40-4.

Referring to Figs. 14 and 15, distal-most position 40-7 of slider body 40 is a position distal to home position 40-4, and is the position where a firing (de-priming) of inner cannula assembly 48, including cutting cannula 58, has occurred, i.e., to sever a tissue sample received into elongate side opening 52-5 of outer cannula 52. As shown in Figs. 14 and 15, slider body 40 includes a depression feature 40-8 configured such that when slider body 40 is moved distally from home position 40-4 toward distal-most position 40-7, depression feature 40-8 engages latch head 54-6 of proximal latch mechanism 54-2 of inner cannula assembly 48 and forces latch head 54-6 of proximal latch mechanism 54-2 downwardly to release distal contact of latch head 54-6 with interior wall 30 at cannula latch opening 30-4. As such, cannula drive spring 60 is released (i.e., fired, or de-primed; see Fig. 7) from the compressed state to propel inner cannula assembly 48, including cutting cannula 58, in the distal direction D1, such that distal cutting edge 58-2 of cutting cannula travels past elongate side opening 52-5 of outer cannula 52 to sever a tissue sample previously received into elongate side opening 52-5 of outer cannula 52 and capture the tissue sample within cannula assembly 16.

As this time, cannula assembly 16 of biopsy device 10 is removed from the patient. The inner cannula assembly 48 having cutting cannula 58 is then retracted to remove the captured tissue sample from elongate side opening 52-5 of outer cannula 52.

If a further sample from this same patient is desired, then the process described above may be repeated.

Following acquisition of all desired samples from the patient, it is recommended that biopsy device 10 be disposed of in its entirety in a safe manner.

While this invention has been described with respect to at least one embodiment, those skilled in the art will recognize that the present invention may be farther modified within the scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A biopsy device (10), comprising:
a housing body (20) defining a longitudinal axis;
a first cannula assembly (46) having a first elongate cannula (52) that extends along the longitudinal axis, the first elongate cannula having a first side wall (52-3) configured to define a first lumen (52-4) and having an elongate side opening (52-5) that extends through the first side wall;
a second cannula assembly (48) including a second cannula body (54) coupled to a second elongate cannula (58) that is slidably received in the first lumen of the first elongate cannula,
the second cannula body having a drive tab (54-1) and a proximal latch mechanism (54-2) configured to releasably latch the second elongate cannula in a retracted position,
the second elongate cannula having a second side wall (58-3) having a second lumen (58-4), and having a vacuum side port (58-5) that extends through the second side wall and is in fluid communication with the second lumen, and
a cannula drive spring (60) configured to compress when the proximal latch mechanism is moved to the retracted position and configured to decompress to propel the second cannula assembly in a distal direction when the proximal latch mechanism is released from the retracted position;
a vacuum source (18) positioned in the housing body, the vacuum source configured to store a vacuum, the vacuum source including a chamber side wall (76) defining a volume (76-2), the chamber side wall having a chamber vacuum port (76-3), and having a vacuum seal (78) interposed in sealing engagement between the chamber vacuum port and the second elongate cannula; and
a trigger slide assembly (14) having a slider body (40) coupled to the housing body and coupled to the drive tab of the second cannula body, and configured such that:
a first proximal movement of the slider body in a proximal direction retracts the second cannula assembly a first distance to latch the proximal latch mechanism of the second cannula assembly in the retracted position and compress the cannula drive spring; and **characterised in that**
a second proximal movement of the slider body in the proximal direction retracts the second cannula assembly a second distance cumulative with the first distance to radially align the vacuum side port of the second elongate cannula with the chamber vacuum port of the vacuum source to supply vacuum from the vacuum source to the second lumen of the second elongate cannula; and
a third movement of the slider body releases the proximal latch mechanism such that the cannula drive spring decompresses to propel the second elongate cannula of the second cannula assembly in the distal direction.

2. The biopsy device of claim 1, wherein the third movement of the slider body is in the distal direction opposite the proximal direction.

3. The biopsy device of claim 1 or 2, wherein the chamber side wall defines a U-shaped volume (76-2), wherein a portion of the chamber side wall defines an elongate recessed trough (80) having a longitudinal extent, and wherein the second elongate cannula is positioned within the elongate recessed trough external to the chamber side wall and without contacting the chamber side wall.

4. The biopsy device of claim 1 or 2, wherein the chamber side wall of the vacuum source has a perimeter (76-1) defining a U-shaped area in cross-section that extends longitudinally to define a U-shaped volume (76-2), and comprising a vacuum plunger mechanism (82) having a U-shaped piston (84) that is positioned in the U-shaped volume, and a chamber seal (85) configured for sealing engagement between an interior surface (76-8) of the chamber side wall (76) and the U-shaped piston.

5. The biopsy device of claim 4, wherein:
the housing body includes an interior wall (30) having a piston latch opening (30-3); and
the vacuum plunger mechanism includes a piston latch mechanism (84-3) that extends from a proximal surface (84-1) of the U-shaped piston (84), the piston latch mechanism configured to pass through the piston latch opening (30-3) of the interior wall and releasably latch the U-shaped piston in a retracted position when the U-shaped piston is moved in the proximal direction to the retracted position.

6. The biopsy device of claim 5, comprising:
a pair of piston link rods (86, 88) that extend proximally from the proximal surface (84-1) of the U-shaped piston, each of the pair of piston link rods having a proximal end (86-1, 88-1) and a bore (86-2, 88-2) extending distally from the proximal end;
a vacuum prime handle mechanism (62) having a handle base (64) and
a pair of elongate handle links (66, 68) that extend from the handle base; and
a pair of joining features (66-1, 68-1) configured to connect the pair of piston link rods to the pair of elongate handle links in sliding engagement.

7. The biopsy device of claim 1 or 2, wherein the chamber side wall has a perimeter that defines an elongate recessed trough (80) having a longitudinal extent, and wherein the second elongate cannula is positioned within the elongate recessed trough external to the chamber side wall and without contacting the chamber side wall.

8. The biopsy device of claim 7, wherein the chamber vacuum port extends outwardly from the chamber side wall from within the elongate recessed trough and in a direction toward the second elongate cannula.

9. The biopsy device of claim 8, wherein the first elongate cannula is stationary with respect to the housing body, and the second elongate cannula is movable within the first lumen relative to the housing body, the second lumen of the second elongate cannula being in fluid communication with the elongate side opening of the first elongate cannula.

10. The biopsy device of claim 1 or 2, comprising a cannula support rod (44) affixed to the housing body, and being coaxial with the first elongate cannula and the second elongate cannula, the cannula support rod being received in the second lumen of the second elongate cannula.

11. The biopsy device of claim 1 or 2, wherein the chamber side wall (76) has a perimeter (76-1) defining a U-shaped area in cross-section, and the chamber side wall extends longitudinally between a chamber open end (72) and a chamber end wall (74) of the vacuum source to define a U-shaped volume (76-2).

## Patentansprüche

1. Biopsievorrichtung (10), umfassend:
einen Gehäusekörper (20), der eine Längsachse definiert;
eine erste Kanülenanordnung (46), die eine erste längliche Kanüle (52) aufweist, die sich entlang der Längsachse erstreckt, wobei die erste längliche Kanüle eine erste Seitenwand (52-3) aufweist, die konfiguriert ist, um ein erstes Lumen (52-4) zu definieren und eine längliche Seitenöffnung (52-5) aufweist, die sich durch die erste Seitenwand erstreckt;
eine zweite Kanülenanordnung (48), die einen zweiten Kanülenkörper (54), der an eine zweite längliche Kanüle (58) gekoppelt ist, der verschiebbar in dem ersten Lumen der ersten länglichen Kanüle aufgenommen ist, wobei der zweite Kanülenkörper eine Stelllasche (54-1) und einen proximalen Verriegelungsmechanismus (54-2) aufweist, der konfiguriert ist, um die zweite längliche Kanüle in einer zurückgezogenen Position lösbar zu verriegeln, wobei die zweite längliche Kanüle eine zweite Seitenwand (58-3) aufweist, die ein zweites Lumen (58-4) aufweist und einen Vakuumseitenanschluss (58-5) aufweist, der sich durch die zweite Seitenwand erstreckt und in Fluidkommunikation mit dem zweiten Lumen steht, und eine Kanülenstellfeder (60), die konfiguriert ist, um zusammengedrückt zu werden, wenn der proximale Verriegelungsmechanismus zu der zurückgezogenen Position bewegt wird, und konfiguriert ist, um sich zu entkomprimieren, um die zweite Kanülenanordnung in einer distalen Richtung vorwärtszutreiben, wenn der proximale Verriegelungsmechanismus aus der zurückgezogenen Position gelöst wird, beinhaltet;
eine Vakuumquelle (18), die in dem Gehäusekörper positioniert ist, wobei die Vakuumquelle konfiguriert ist, um ein Vakuum zu speichern, wobei die Vakuumquelle eine Kammerseitenwand (76) beinhaltet, die ein Volumen (76-2) definiert, wobei die Kammerseitenwand einen Kammervakuumanschluss (76-3) aufweist und eine Vakuumdichtung (78) aufweist, die zwischen dem Kammervakuumanschluss und der zweiten länglichen Kanüle in einem Abdichtungseingriff eingefügt ist; und
eine Auslöseverschiebeanordnung (14), die einen Schiebekörper (40) aufweist, der an den Gehäusekörper gekoppelt ist und an die Stelllasche des zweiten Kanülenkörpers gekoppelt ist und derart konfiguriert ist, dass:
eine erste proximale Bewegung des Schiebekörpers in einer proximalen Richtung die zweite Kanülenanordnung um eine erste Distanz zur Verriegelung des proximalen Verriegelungsmechanismus der zweiten Kanülenanordnung in der zurückgezogenen Position zurückzieht und die Kanülenstellfeder zusammendrückt; und
**dadurch gekennzeichnet, dass**
eine zweite proximale Bewegung des Schiebekörpers in der proximalen Richtung die zweite Kanülenanordnung um eine zweite Distanz kumulativ zu der ersten Distanz zurückzieht, um den Vakuumseitenanschluss der zweiten länglichen Kanüle mit dem Kammervakuumanschluss der Vakuumquelle radial auszurichten, um Vakuum von der Vakuumquelle an das zweite Lumen der zweiten länglichen Kanüle bereitzustellen; und
eine dritte Bewegung des Schiebekörpers den proximalen Verriegelungsmechanismus derart löst, dass sich die Kanülenstellfeder entkomprimiert, um die zweite längliche Kanüle der zweiten Kanülenanordnung in der distalen Richtung vorwärtszutreiben.

2. Biopsievorrichtung nach Anspruch 1, wobei die dritte Bewegung des Schiebekörpers in der distalen Richtung, entgegengesetzt der proximalen Richtung, verläuft.

3. Biopsievorrichtung nach Anspruch 1 oder 2, wobei die Kammerseitenwand ein U-förmiges Volumen (76-2) definiert, wobei ein Abschnitt der Kammerseitenwand eine längliche vertiefte Rinne (80) definiert, die eine Längserstreckung aufweist, und wobei die zweite längliche Kanüle innerhalb der länglichen vertieften Rinne extern der Kammerseitenwand positioniert ist, ohne die Kammerseitenwand zu berühren.

4. Biopsievorrichtung nach Anspruch 1 oder 2, wobei die Kammerseitenwand der Vakuumquelle einen Umfang (76-1) aufweist, der einen U-förmigen Bereich in Querschnitt definiert, der sich in Längsrichtung erstreckt, um ein U-förmiges Volumen (76-2) zu definieren, und einen Vakuumkolbenmechanismus (82), der einen U-förmigen Hubkolben (84) aufweist, der in dem U-förmigen Volumen positioniert ist, und eine Kammerabdichtung (85), die für einen Abdichtungseingriff zwischen einer Innenoberfläche (76-8) der Kammerseitenwand (76) und dem U-förmigen Hubkolben konfiguriert ist, umfasst.

5. Biopsievorrichtung nach Anspruch 4, wobei:
der Gehäusekörper eine Innenwand (30) einschließt, die eine Hubkolbenverriegelungsöffnung (30-3) aufweist; und
der Vakuumkolbenmechanismus einen Hubkolbenverriegelungsmechanismus (84-3) einschließt, der sich von einer proximalen Oberfläche (84-1) des U-förmigen Hubkolbens (84) erstreckt, wobei der Hubkolbenverriegelungsmechanismus konfiguriert ist, um durch die Hubkolbenverriegelungsöffnung (30-3) der Innenwand hindurchzugehen und den U-förmigen Hubkolben in einer zurückgezogenen Position lösbar zu verriegeln, wenn der U-förmige Hubkolben in der proximalen Richtung zu der zurückgezogenen Position bewegt wird.

6. Biopsievorrichtung nach Anspruch 5, umfassend:
ein Paar von Hubkolbenverbindungsstangen (86, 88), die sich proximal von der proximalen Oberfläche (84-1) des U-förmigen Hubkolbens erstrecken, wobei jede des Paars von Hubkolbenverbindungsstangen ein proximales Ende (86-1, 88-1) und eine Bohrung (86-2, 88-2) aufweist, die sich distal von dem proximalen Ende erstrecken;
einen Vakuumvorbereitungsgriffmechanismus (62), der eine Griffbasis (64) und ein Paar von länglichen Griffverbindungen (66, 68) aufweist, die sich von der Griffbasis erstrecken; und
ein Paar von Fügeeigenschaften (66-1, 68-1), die konfiguriert sind, um das Paar von Hubkolbenverbindungsstangen mit dem Paar von länglichen Griffverbindungen in einem Verschiebeeingriff zu verbinden.

7. Biopsievorrichtung nach Anspruch 1 oder 2, wobei die Kammerseitenwand einen Umfang aufweist, der eine längliche vertiefte Rinne (80) definiert, die eine Längserstreckung aufweist, und wobei die zweite längliche Kanüle innerhalb der länglichen vertieften Rinne extern der Kammerseitenwand positioniert ist, ohne die Kammerseitenwand zu berühren.

8. Biopsievorrichtung nach Anspruch 7, wobei sich der Kammervakuumanschluss nach außen von der Kammerseitenwand von innerhalb der länglichen Vertiefung aus durch und in einer Richtung zu der zweiten länglichen Kanüle hin erstreckt.

9. Biopsievorrichtung nach Anspruch 8, wobei die erste längliche Kanüle in Bezug auf den Gehäusekörper stationär ist und die zweite längliche Kanüle innerhalb des ersten Lumens relativ zum Gehäusekörper bewegbar ist, wobei das zweite Lumen der zweiten länglichen Kanüle in Fluidkommunikation mit der länglichen Seitenöffnung der ersten länglichen Kanüle ist.

10. Biopsievorrichtung nach Anspruch 1 oder 2, umfassend eine Kanülenstützstange (44), die am Gehäusekörper angebracht ist und koaxial mit der ersten länglichen Kanüle und der zweiten länglichen Kanüle ist, wobei die Kanülenstützstange im zweiten Lumen der zweiten länglichen Kanüle aufgenommen wird.

11. Biopsievorrichtung nach Anspruch 1 oder 2, wobei die Kammerseitenwand (76) einen Umfang (76-1) aufweist, der einen U-förmigen Bereich in Querschnitt definiert und sich die Kammerseitenwand in Längsrichtung zwischen einem offenen Kammerende (72) und einer Kammerendwand (74) der Vakuumquelle erstreckt, um ein U-förmiges Volumen (76-2) zu definieren.

## Revendications

1. Dispositif de biopsie (10), comprenant :
un corps de boîtier (20) définissant un axe longitudinal ;
un premier ensemble canule (46) ayant une première canule allongée (52) qui s'étend le long de l'axe longitudinal, la première canule allongée ayant une première paroi latérale (52-3) configurée pour définir une première lumière (52-4) et ayant une ouverture latérale allongée (52-5) qui s'étend à travers la première paroi latérale ;
un second ensemble canule (48) incluant un second corps de canule (54) couplé à une seconde canule allongée (58) qui est reçue de manière coulissante dans la première lumière de la première canule allongée,
le second corps de canule ayant une patte d'entraînement (54-1) et un mécanisme de verrouillage proximal (54-2) configuré pour verrouiller de façon amovible la seconde canule allongée dans une position rétractée, la seconde canule allongée ayant une seconde paroi latérale (58-3) ayant une seconde lumière (58-4), et ayant un orifice côté vide (58-5) qui s'étend à travers la seconde paroi latérale et est en communication fluidique avec la seconde lumière, et
un ressort d'entraînement de canule (60) configuré pour se comprimer lorsque le mécanisme de verrouillage proximal est déplacé jusqu'à la position rétractée et configuré pour se décomprimer pour propulser le second ensemble canule dans un sens distal lorsque le mécanisme de verrouillage proximal est libéré de la position rétractée ;
une source de vide (18) positionnée dans le corps de boîtier, la source de vide étant configurée pour stocker un vide, la source de vide incluant une paroi latérale de chambre (76) définissant un volume (76-2), la paroi latérale de chambre ayant un orifice à vide de chambre (76-3), et ayant un joint de vide (78) interposé dans un engagement d'étanchéité entre l'orifice à vide de chambre et la seconde canule allongée ; et
un ensemble coulisseau de déclenchement (14) ayant un corps de coulisseau (40) couplé au corps de boîtier et couplé à la patte d'entraînement du second corps de canule, et configuré de sorte que :
un premier mouvement proximal du corps de coulisseau dans un sens proximal rétracte le second ensemble canule sur une première distance pour verrouiller le mécanisme de verrouillage proximal du second ensemble canule dans la position rétractée et pour comprimer le ressort d'entraînement de canule ; et **caractérisé en ce que**
un deuxième mouvement proximal du corps de coulisseau dans le sens proximal rétracte le second ensemble canule sur une seconde distance cumulative avec la première distance pour aligner de façon radiale l'orifice côté vide de la seconde canule allongée avec l'orifice à vide de chambre de la source de vide pour fournir un vide à partir de la source de vide jusqu'à la seconde lumière de la seconde canule allongée ; et un troisième mouvement du corps de coulisseau libère le mécanisme de verrouillage proximal de sorte que le ressort d'entraînement de canule se décomprime pour propulser la seconde canule allongée du second ensemble canule dans le sens distal.

2. Dispositif de biopsie selon la revendication 1, dans lequel le troisième mouvement du corps de coulisseau est dans le sens distal opposé au sens proximal.

3. Dispositif de biopsie selon la revendication 1 ou 2, dans lequel la paroi latérale de chambre définit un volume en forme de U (76-2), dans lequel une portion de la paroi latérale de chambre définit un creux évidé allongé (80) ayant une étendue longitudinale, et dans lequel la seconde canule allongée est positionnée à l'intérieur du creux évidé allongé extérieur à la paroi latérale de chambre et sans venir en contact avec la paroi latérale de chambre.

4. Dispositif de biopsie selon la revendication 1 ou 2, dans lequel la paroi latérale de chambre de la source de vide a un périmètre (76-1) définissant une zone en forme de U en section transversale qui s'étend longitudinalement pour définir un volume en forme de U (76-2), et
comprenant un mécanisme de plongeur à vide (82) ayant un piston en forme de U (84) qui est positionné dans le volume en forme de U, et un joint de chambre (85) configuré pour un engagement d'étanchéité entre une surface intérieure (76-8) de la paroi latérale de chambre (76) et le piston en forme de U.

5. Dispositif de biopsie selon la revendication 4, dans lequel :
le corps de boîtier inclut une paroi intérieure (30) ayant une ouverture de verrouillage de piston (30-3) ; et
le mécanisme de plongeur à vide inclut un mécanisme de verrouillage de piston (84-3) qui s'étend à partir d'une surface proximale (84-1) du piston en forme de U (84), le mécanisme de verrouillage de piston étant configuré pour passer à travers l'ouverture de verrouillage de piston (30-3) de la paroi intérieure et pour verrouiller de façon amovible le piston en forme de U dans une position rétractée lorsque le piston en forme de U est déplacé dans le sens proximal jusqu'à la position rétractée.

6. Dispositif de biopsie selon la revendication 5, comprenant :
une paire de bielles de piston (86, 88) qui s'étend de façon proximale à partir de la surface proximale (84-1) du piston en forme de U, chacune de la paire de bielles de piston ayant une extrémité proximale (86-1, 88-1) et un alésage (86-2, 88-2) s'étendant de façon distale à partir de l'extrémité proximale ;
un mécanisme de poignée d'amorçage de vide (62) ayant une base de poignée (64) et une paire de liaisons de poignée allongées (66, 68) qui s'étendent à partir de la base de poignée ; et
une paire d'éléments de jonction (66-1, 68-1) configurée pour raccorder la paire de bielles de piston à la paire de liaisons de poignée allongées dans un engagement coulissant.

7. Dispositif de biopsie selon la revendication 1 ou 2, dans lequel la paroi latérale de chambre a un périmètre qui définit un creux évidé allongé (80) ayant une étendue longitudinale, et dans lequel la seconde canule allongée est positionnée à l'intérieur du creux évidé allongé extérieur à la paroi latérale de chambre et sans venir en contact avec la paroi latérale de chambre.

8. Dispositif de biopsie selon la revendication 7, dans lequel l'orifice à vide de chambre s'étend vers l'extérieur à partir de la paroi latérale de chambre depuis l'intérieur du creux évidé allongé et dans un sens vers la seconde canule allongée.

9. Dispositif de biopsie selon la revendication 8, dans lequel la première canule allongée est stationnaire par rapport au corps de boîtier, et la seconde canule allongée est mobile à l'intérieur de la première lumière par rapport au corps de boîtier, la seconde lumière de la seconde canule allongée étant en communication fluidique avec l'ouverture latérale allongée de la première canule allongée.

10. Dispositif de biopsie selon la revendication 1 ou 2, comprenant une tige de support de canule (44) fixée au corps de boîtier et étant coaxiale avec la première canule allongée et la seconde canule allongée, la tige de support de canule étant reçue dans la seconde lumière de la seconde canule allongée.

11. Dispositif de biopsie selon la revendication 1 ou 2, dans lequel la paroi latérale de chambre (76) a un périmètre (76-1) définissant une zone en forme de U en section transversale, et la paroi latérale de chambre s'étend longitudinalement entre une extrémité ouverte de chambre (72) et une paroi d'extrémité de chambre (74) de la source de vide pour définir un volume en forme de U (76-2).
